# EUROPEAN PATENT APPLICATION

(11) **EP 2 250 972 A1**
(43) Date of publication of application: **17.11.2010**
(21) Application number: 09173173.7
(22) Date of filing: 15.10.2009
(51) Int. Cl.: A61C 8/00

(54) **Surgical apparatus, particularly of the dental type, for procedures for raising the maxillary sinus**

(30) Priority: 15.05.2009 IT PD20090136
(71) Applicant: Spano Technology S.r.l., 35020 ALBIGNASEGO PD (IT)
(72) Inventor: Cohen, Simon, 44122, Ferrara (IT); Spano, Ernesto, 30174, Zelarino VE (IT)
(74) Representative: Gallo, Luca

(57) **Abstract**

A surgical apparatus (10), particularly of the dental type for procedures for raising the maxillary sinus, which comprises
- an elongated body (11),
- a working head (12) which is supported by a first end (11a) of the elongated body (11), so that it can rotate about a work axis (A), the work axis (A) being substantially transverse to the longitudinal extension (B) of the elongated body (11),
- a device (13) for moving the head (12), which is supported by the elongated body (11) and comprises at least one element (14) for the actuation of the head (12), which is spaced from the head (12) and is functionally connected thereto for allowing the user to operate on the actuation element (14) from the outside of a mouth within which the head (12) is positioned during use,
- a percussion device (15), which is supported by the elongated body (11) and is adapted to impart, by means of the head (12), when actuated, force impulses to a work region.

## Description

The present invention relates to a surgical apparatus, particularly of the dental type, for procedures for raising the maxillary sinus.

Many tools are currently used to raise the maxillary sinus.

The motorized turbine device commonly used in dental treatment procedures in dentistry, whose working head is equipped with a milling cutter, is used to mill the bone part.

Due to the high rotation rate of the working head, and therefore of the milling cutter, there is however the risk of overheating of the region being worked, and therefore continuous wetting with water is required.

However, this wetting washes away the blood, which instead advantageously should not be removed in order to facilitate spontaneous healing of the wound.

In order to overcome these drawbacks, currently it is known to use a manual mill.

Such mill has a milling cutter fitted on a transverse handpiece.

By pressing the milling cutter on the bone and turning it around its own axis, rotating the handpiece repeatedly, the operator perforates the bone part gradually and in a controlled manner.

However, the use of this tool is awkward due to the narrow maneuvering space offered by the mouth of the patient.

Moreover, since the operation of turning the handpiece is awkward, during this operation the operator can easily offset the milling cutter axially with respect to the hole being formed, damaging its walls, to the full disadvantage of precision in work.

After the milling, in the treatment for lifting the maxillary sinus tools are used that are composed of an elongated and thin handpiece, at the end of which a tool head is fitted axially which, depending on the work to be performed, is a bone expander, a sinus lifter for lifting the sinus, a bone compactor or condenser, for compacting the thickening filler of the maxillary sinus.

In particular, for raising the sinoidal sinus a hammer is used to strike the free end of the handpiece while the end fitted with the sinus lifter is inserted in the hole.

This operation is awkward, since the handpiece must be inserted transversely in the mouth for alignment of the same with the axis of the hole and must be struck in a strictly axial direction in order to avoid damage to the maxillary bone, further damaging the provided hole, to the full detriment of the stability of the dental implant that will be inserted therein.

This operation is notoriously very complicated to perform correctly and effectively, since it requires considerable sensitivity of the operator, who as mentioned must not axially offset the lifter with respect to the hole, so as to not damage it, and most of all must not impart an excessive impulse to the lifter to avoid the piercing of the Schneider membrane.

In the field of dentistry, the need is therefore strongly felt to have tools that make it possible to easily perform operations for lifting the maxillary sinus safely and effectively.

The aim of the present invention is to meet this need, by providing a surgical apparatus, particularly of the dental type for procedures for raising the maxillary sinus, which makes it possible to mill the maxillary bone more easily than currently known devices and tools, simultaneously allowing safe control of the operation by the operator.

Within this aim, an object of the invention is to provide a surgical apparatus that makes it possible to easily raise the perforated maxillary sinus, allowing great sensitivity of the operator in order to avoid damage to the patient.

Another object of the invention is to provide a surgical apparatus that allows easy perforation of the maxillary bone by milling, making it possible to actuate the mill from outside the mouth.

Another object of the invention is to provide a surgical apparatus that allows easier and swifter compaction of the thickening filler of the maxillary sinus than with the use of currently known compactors.

Another object of the invention is to provide a surgical apparatus which is structurally simple and easy to use and can be manufactured at low cost.

This aim, as well as these and other objects that will become better apparent hereinafter, are achieved by a surgical apparatus, particularly of the dental type for procedures for raising the maxillary sinus, **characterized in that** it comprises
- an elongated body,
- a working head which is supported by a first end of said elongated body, so that it can rotate about a work axis, said work axis being substantially transverse to the longitudinal extension of said elongated body,
- a device for moving said head, which is supported by said body and comprises at least one element for the actuation of said head, which is spaced from said head and is functionally connected thereto for allowing the user to operate on said actuation element from the outside of a mouth within which said head is placed during use,
- a percussion device, which is supported by said body and is adapted to impart, by means of said head, when actuated, force impulses to a work region.

Further characteristics and advantages of the invention will become better apparent from the following detailed description of a preferred but not exclusive embodiment of the surgical apparatus according to the invention, illustrated by way of non-limiting example in the accompanying drawings, wherein:

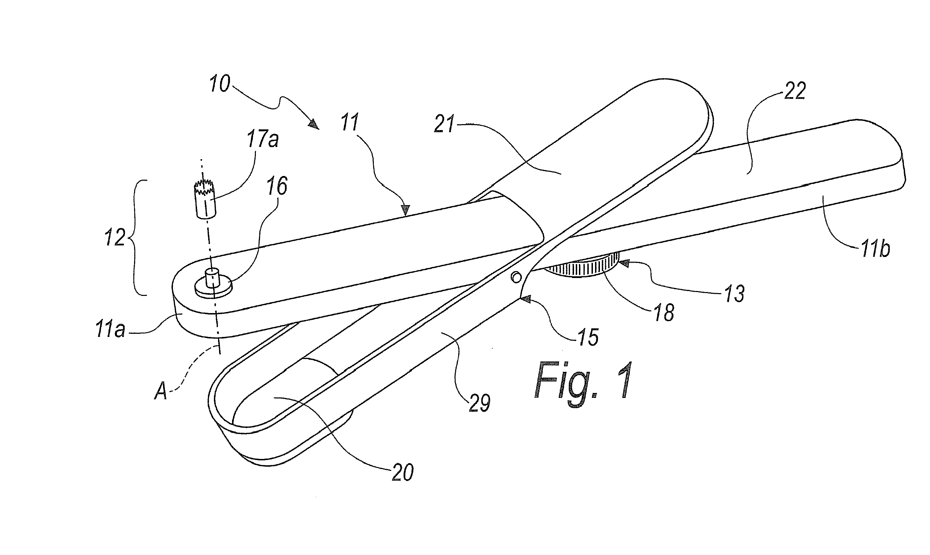

Figure 1 is a perspective view of a surgical apparatus according to the invention;
Figures 2 and 3 are side elevation views of the surgical apparatus according to the invention in two different configurations;
Figure 4 is a top plan view of the surgical apparatus according to the invention.

It is noted that anything found to be already known during the patenting process is understood not to be claimed and to be the subject of a disclaimer.

With reference to the figures, the reference numeral 10 generally designates a surgical apparatus, particularly of the dental type for procedures for raising the maxillary sinus, which has a particularity in that it comprises
- an elongated body 11,
- a working head 12 that is supported by a first end 11a of the elongated body 11, so that it can rotate about a work axis A, the work axis A being substantially transverse to the longitudinal extension B of the elongated body 11,
- a device 13 for moving the head 12, which is supported by the elongated body 11 and comprises an element 14 for the actuation of the head 12,
- a percussion device 15, which is supported by the elongated body 11 and is adapted to impart, by means of the head 12, when actuated, force impulses to a work region.

The actuation element 14 is spaced from the head 12 and is functionally connected thereto, so as to allow the user to operate on the actuation element 14 from the outside of a mouth within which the head 12 is positioned during use.

Advantageously, the head 12 comprises a universal coupling 16 and at least one interchangeable head-tool 17.

Conveniently, the head-tool 17 is selected among milling cutters 17a, chisels 17b, sinus lifters 17c, bone condensers 17d and similar tools for raising the maxillary sinus.

More particularly, the actuation element 14 comprises a knob 18 that is rotatably supported by the elongated body 11, the movement device 13 comprising a mechanical transmission 19 for the functional connection of the knob 18 to the head 12, for the transmission of a working torque.

Conveniently, the rotation axis C of the knob 18 is substantially parallel to the work axis A.

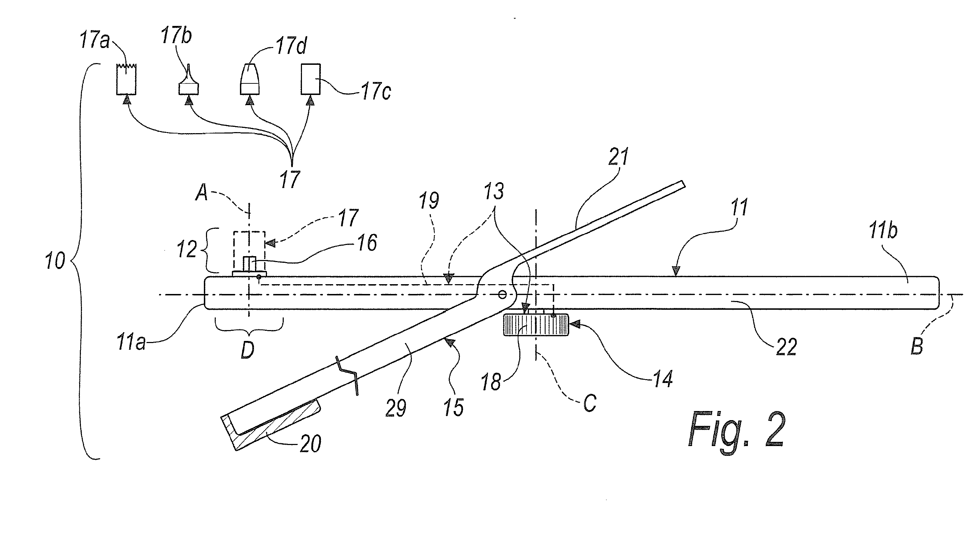

The mechanical transmission 19, shown schematically in Figure 2 by means of a dashed line and not shown further in the accompanying figures, advantageously is provided as a series of gears and can be provided in a manner which is substantially equivalent to a chain or belt transmission that is appropriately provided with teeth so as to transfer effectively the torque applied to the knob 18 by the operator to the head 12.

The percussion device 15 preferably comprises a rocker 29, which is hinged in an oscillating manner to the elongated body 11 and has a percussion element 20 at one end that corresponds to the first end 11a and an actuation lever 21 at the opposite end, which is arranged toward the second end 11b of the elongated body 11.

Advantageously, the surgical apparatus 10 is made of a material that can be easily sterilized and washed, such as stainless steel.

Further, the second end 11b of the body 11 has a portion that is elongated so as to form a handpiece 22, which in embodiments that are not shown in the accompanying figures can have an ergonomic shape or an ergonomic covering adapted to ensure stable and safe grip.

The use of a surgical apparatus according to the invention is as follows.

For milling, the universal coupling 16 of the head 12 is provided by connecting thereto a milling cutter 17a.

The operator then performs milling by arranging the milling cutter 17a in the work region and actuating it by means of the knob 18, which is external to the mouth of the patient, to the full advantage of easy actuation.

Once milling has ended, for raising the maxillary sinus the head 12 is again provided with a tool by associating a sinus lifter 17c with the universal coupling 16.

In use, the force impulses to be imparted by means of the sinus lifter 17c in the work area are provided by the user by making the rocker 29 oscillate, by operating on the lever 21, so as to move the percussion element 20 into abutment against an impact region D of the first end 11a that lies opposite the head 12.

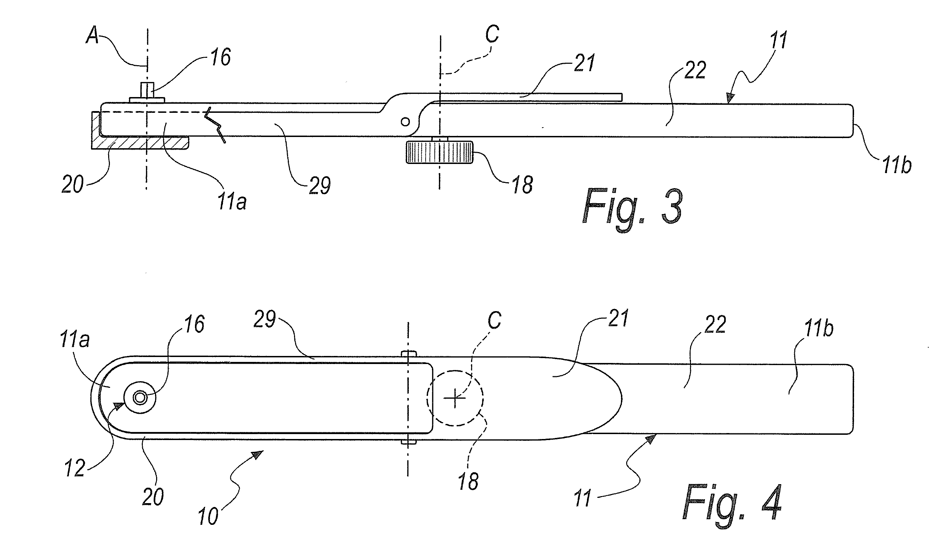

Said impulses are for example provided by making the surgical apparatus 10 pass from a configuration such as the one shown in Figure 2 to a configuration such as the one shown in Figure 3.

Likewise, for compaction, the head 12 is equipped with a bone condenser 17d and the work area is compacted by imparting thereto, by means of such condenser, force impulses generated as mentioned.

In practice it has been found that the invention achieves the intended aim and objects, providing a surgical apparatus, particularly of the dental type for procedures for raising the maxillary sinus, that makes it possible to mill the maxillary bone more easily than currently known devices and equipment, operating the mill from outside of the mouth by means of the knob, simultaneously allowing safe control of operation by the operator, who has an easy and safe grip on the apparatus, by gripping it by its handle, by means of which he is further able to impart a milling cutter penetration force that can be modulated easily.

A surgical apparatus according to the invention further makes it possible to lift the perforated maxillary sinus easily, allowing great sensitivity of the operator; in order to avoid patient injury, the operator is in fact able to modulate easily the intensity of the lifting impulses, generating pulses that are controlled by means of the oscillation of the rocker, which makes the percussion unit abut against the first end of the body of the apparatus.

A surgical apparatus according to the invention further allows an easier and quicker compaction of the filler for thickening the maxillary sinus than by using currently known compactors, the rocker being easy to operate by means of the lever that is arranged outside the mouth and is directed toward the operator.

Moreover, a surgical apparatus according to the invention is structurally simple and can be manufactured at low cost.

The invention thus conceived is susceptible of numerous modifications and variations, all of which are within the scope of the appended claims; all the details may further be replaced with other technically equivalent elements.

In practice, the materials employed, as long as they are compatible with the specific use, as well as the contingent shapes and dimensions, may be any according to requirements and to the state of the art.

The disclosures in Italian Patent Application No. PD2009A000136 from which this application claims priority are incorporated herein by reference.

Where technical features mentioned in any claim are followed by reference signs, those reference signs have been included for the sole purpose of increasing the intelligibility of the claims and accordingly such reference signs do not have any limiting effect on the interpretation of each element identified by way of example by such reference signs.

## Claims

1. A surgical apparatus (10), particularly of the dental type for procedures for raising the maxillary sinus, **characterized in that** it comprises
- an elongated body (11),
- a working head (12) which is supported by a first end (11a) of said elongated body (11), so that it can rotate about a work axis (A), said work axis (A) being substantially transverse to the longitudinal extension (B) of said elongated body (11),
- a device (13) for moving said head (12), which is supported by said elongated body (11) and comprises at least one element (14) for the actuation of said head (12), which is spaced from said head (12) and is functionally connected thereto for allowing the user to operate on said actuation element (14) from the outside of a mouth within which said head (12) is positioned during use,
- a percussion device (15), which is supported by said elongated body (11) and is adapted to impart, by means of said head (12), when actuated, force impulses to a work region.

2. The surgical apparatus according to claim 1, **characterized in that** said head (12) comprises a universal coupling (16) and at least one interchangeable tool-head (17).

3. The surgical apparatus according to claim 2, **characterized in that** said at least one tool-head (17) is selected among milling cutters (17a), chisels (17b), sinus lifters (17c), bone condensers (17d) and similar tools for raising the maxillary sinus.

4. The surgical apparatus according to one or more of the preceding claims, **characterized in that** said at least one actuation element (14) comprises a knob (18) that is supported rotatably by said elongated body (11), said movement device (13) comprising a mechanical transmission (19) for the functional connection of said knob (18) to said head (12), for transmission of a working torque.

5. The surgical apparatus according to one or more of the preceding claims, **characterized in that** said percussion device (15) comprises a rocker (29), which is hinged so that it can oscillate with respect to said elongated body (11) and has a percussion element (20) at one end which corresponds to said first end (11a) and an actuation lever (21), at the opposite end, which is arranged toward the second end (11b) of said elongated body (11).
